# EUROPEAN PATENT SPECIFICATION

(11) **EP 1 894 562 B1**
(45) Date of publication and mention of the grant of the patent: **15.12.2010**
(21) Application number: 07121574.3
(22) Date of filing: 15.08.2003
(51) Int. Cl.: A61K 9/50

(54) **Pharmaceutical compositions comprising an opioid antagonist**
Pharmazeutische Zusammensetzungen enthaltend einen Opioidantagonisten
Compositions pharmaceutiques comprenant un antagoniste des récepteurs opiacés

(30) Priority: 15.08.2002 US 403711 P
(43) Date of publication of application: 05.03.2008
(62) Divisional of application: 03751860.2
(73) Proprietor: EURO-CELTIQUE S.A., 1653 Luxembourg (LU)
(72) Inventor: Oshlack, Benjamin, Boca Raton, FL 33432 (US); Huang, Hua-Pin, Englewood Cliffs, NJ 07726 (US); Gullapalli, Rampurna, San Bruno, CA 94066 (US); Machonis, Meredith, Bridgewater, New Jersey 08807 (US)
(74) Representative: Glas, Holger

(56) References cited:
- WO-A-01/58447
- WO-A-01/85257
- WO-A-03/013479
- WO-A-03/077867
- US-A1- 2002 010 127

## Description

### FIELD OF THE INVENTION

The present invention relates to pharmaceutical substrate compositions comprising a therapeutically active agent, a diffusion barrier coating comprising an anionic polymer and a coating comprising a hydrophobic material coated over said diffusion barrier coating.

### BACKGROUND OF THE INVENTION

It is known in the pharmaceutical art to prepare compositions which provide for controlled release of pharmacologically active substances contained in the compositions after oral administration to humans and animals. Controlled release formulations known in the art include specially coated pellets, coated tablets and capsules, and ion exchange resins, wherein the slow release of the active medicament is brought about through selective breakdown of the coating of the preparation or through compounding with a special matrix to affect the release of a drug. Some controlled release formulations provide for related sequential release of a single dose of an active compound at predetermined periods after administration.

One of the requirements for an acceptable pharmaceutical composition is that it must be stable, so as not to exhibit substantial decomposition of the active ingredient during the time between manufacture of the composition and use by the patient.

In certain instances, it has been found that certain active ingredients may tend to leak or seep through the coatings of certain dosage forms during the manufacturing process which may result in the immediate release of the active agent upon administration when a controlled release of the active agent is desired. Additionally, in certain instances, the leak or seepage of the active agent may result in the substantial release of the active agent where no or substantially no release of the active agent is desired.

WO 01/58447 discloses a controlled release dosage form containing an opioid agonist, an opioid antagonist, and a controlled release material, said dosage form releasing during a dosing interval an analgesic or sub-analgesic amount of the opioid agonist along with an amount of said opioid antagonist effective to attenuate a side effect of said opioid agonist.

There exists a need in the art to develop controlled release pharmaceutical formulations wherein the active ingredient in the formulation does not migrate through the controlled release coating during the manufacturing process and/or upon storage prior to administration of the formulation.

### OBJECTS AND SUMMARY OF THE INVENTION

It is an object of the present invention to provide an oral controlled release pharmaceutical formulation having an improved stability of the therapeutic agent in the formulation by the inclusion of an anionic polymer in the formulation.

It is a further object of certain embodiments of the present invention to provide an oral controlled release pharmaceutical formulation having decreased migration of therapeutic agent through the controlled release coating during the manufacturing process and/or upon storage prior to administration of the formulation.

It is a further object of certain embodiments of the present invention to provide an oral pharmaceutical formulation comprising a substrate having a therapeutic agent, a diffusion barrier coating comprising an anionic polymer coated over the substrate, and a coating comprising a hydrophobic material coated over the diffusion barrier coating.

These objects and others are accomplished by the present invention, which is directed in part to a pharmaceutical formulation comprising a therapeutic agent, a diffusion barrier coating, and a coating comprising a hydrophobic material.

The present invention is directed to a substrate formulation as defined in claim 1.

In certain preferred embodiments, the pharmaceutical formulation comprises a pharmaceutically acceptable inert bead formulation.

In certain embodiments, the coating comprising the hydrophobic material provides for the controlled released of the therapeutic agent.

In certain embodiments, the coating comprising the hydrophobic material provides for the sequestration of the therapeutic agent.

The therapeutic agent is a protonated opioid antagonist e.g., a drug which is positively charged.

The pharmaceutical formulation of the present invention comprises a substrate comprising an opioid antagonist, a diffusion barrier coating comprising an anionic polymer coated over the substrate, and a coating comprising a hydrophobic material coated over said diffusion barrier coating.

In certain embodiments, the pharmaceutical formulation of the present invention comprises a substrate comprising an opioid analgesic, a diffusion barrier coating comprising an anionic polymer coated over the substrate, and a coating comprising a hydrophobic material coated over said diffusion barrier that provides for the controlled release of the opioid analgesic.

For purposes of the present invention, the term "controlled release" means that the therapeutic agent is released from the formulation at a controlled rate such that therapeutically beneficial blood levels (but below toxic levels) of the agent are maintained over an extended period of time, e.g., providing an 8 to 24 hour therapeutic effect.

For purposes of the present invention, the term "sequestered" means the therapeutic agent is not released or not substantially released when the dosage form is administered intact. For example, PCT Publication No. WO 01/58451, discloses an oral dosage form comprising a sequestered opioid antagonist which is not released or substantially not released when the dosage form is administered intact.

### DETAILED DESCRIPTION

The present invention is directed to improving the stability of an oral controlled release pharmaceutical formulation comprising a therapeutic agent by the inclusion of an anionic polymer in the formulation. The formulation of the present invention preferably has three components. The first component is a substrate which comprises one or more therapeutic agents. The therapeutic agent is preferably coated onto the substrate. The second component is an anionic polymer layer, which is coated onto the substrate comprising the therapeutic agent (e.g., coated over the therapeutic agent). The third component is a coating comprising a hydrophobic material and is coated over the second component. The third component may provide for the controlled release of the therapeutic agent or alternatively may provide for the sequestration of the therapeutic agent. The therapeutic agent is a protonated opioid antagonist (e.g., positively charged) and the anionic polymer of the second component, having an affinity for the protonated drug molecule binds with and prevents the diffusion of the therapeutic agent through the hydrophobic coating of the formulation during the manufacturing process and/or upon storage prior to administration. The diffusion of the agent through the hydrophobic coating is especially problematic during the manufacturing process when the hydrophobic material is applied to the substrate as an aqueous dispersion. Accordingly, the diffusion barrier coating is useful in such embodiments to prevent or reduce the migration during the application of the aqueous dispersion of hydrophobic material.

Therapeutic agents for use in the formulations of the present invention are preferably protonated drugs (e.g., positively charged) that have affinity for the anionic polymer in the anionic polymer layer. In certain embodiments, the therapeutic agent of the present invention is a narcotic antagonist (e.g., naltrexone, naloxone, nalorphone) and/or an opiate analgesic (e.g., anileridine, buprenorphine, codeine, fentanyl, hydrocodone, hydromorphone, levorphanol, morphine, meperidine, oxycodone, oxymorphone, trsmadol). In certain alternative embodiments, the therapeutic agent can be selcted from, e.g., cardiovascular drugs (e.g., acebutolol, amiodarone, clonidine, enalapril, guanfacine, hydralazine, mecamylamine, nicardipine, nifenalol, procainamide, quinidine, sotalol, verapamil), antihistamines (e.g., antazoline, bromopheniramine, carbinoxamine, cetirizine, chlorpheniramine, clemastine, diphenhydramine, doxylamine, promethazine), respiratory drugs (e.g., dextromethorphan, pseudoephedrine, albuterol), CNS stimulants (e.g., amphetamine, caffeine methylphenidate, sibutramine), antiviral/antibacterial/antimalarial drugs (e.g, amantadine, amikacin, amodiaquine, bacampicillin, chloroquine, primaquine, quinine), antidepressants (e.g, acepromazine, amitriptyline, bupropion, desipramine, doxepin, fluoxetine, imipramine, nefazodone, nortriptyline, phenelzine, protriptyline, sertraline, trazodone, trimipramine, venlafaxine), anesthetics (e.g, bupivacaine, chloroprocaine, lidocaine, mepivacaine, prilocaine, procaine, tetracaine), CNS depressants (buspirone, chlordiazepoxide, flurazepam, hydroxyzine, midazolam, zolpidem),, mixtures thereof, salts thereof, and the like. In certain embodiments, the agent comprises an opioid antagonist. In certain embodiments, the agent comprises both an opioid analgesic and an opioid antagonist. In preferred embodiments, the drug molecule is in the form of the acidic salt of the drug molecule.

Preferably the therapeutic agent is applied to the substrate. The substrates coated with the therapeutic agent may be prepared, e.g., by dissolving the therapeutic agent in a solvent such as water and then spraying the solution onto the substrates, e.g., nu pariel 18/20 beads. A preferred method of applying the therapeutic agent to the substrate is through the use of a polymer film. An example of a polymer film for use in the present invention includes for example and without limitation, hydroxypropylmethylcellulose, ethylcellulose, polyvinylpyrrolidone, hydroxypropylcellulose, ethylcellulose, mixtures thereof, and the like. The polymer may be dissolved or dispersed in an aqueous or organic medium with the therapeutic agent and coated onto the substrates. In addition to the therapeutic agent, the polymer film may contain optional fillers, pigments, and dyes known in the art.

Conventional coating techniques such as spray or pan coating, as described hereinafter, may be employed to apply the coating comprising the therapeutic agent to the substrate. The amount of the therapeutic agent applied to the substrate may vary depending upon the concentration desired in the finished product. Preferably the amount of the weight of the applied film including the therapeutic agent on the substrate is from about 1 to about 50% weight gain, more preferably from about 2 to about 30 % weight gain.

Substrates for use in the present invention include, for example and without limitation, beads, microspheres, seeds, pellets, ion-exchange resin beads, other multiparticulate systems, and the like. Preferably the substrates of the present invention are pharmaceutically acceptable inert beads. The beads are typically made from one or a mixture of a group selected from but not limited to sucrose, mannitol, lactose, dextrose, sorbitol, cellulose, starch, mixtures thereof, and the like. The preferred size of the inert beads is in the range of from 0.1 mm to about 2.5 mm. The inert beads are preferably pre-manufactured beads known in the art (e.g., non-pareil PG beads). In certain embodiments, the substrates for use in the present invention may include a matrix multiparticulate system, which may comprise the therapeutic agent in a plurality of immediate release matrices, or a compressed matrix formulation (e.g., matrix tablet) comprising the therapeutic agent in an immediate or controlled release matrix.

In accordance with the present invention, the substrates comprising the therapeutic agent, are then overcoated with the diffusion barrier coating. In a preferred embodiment, wherein the substrate is a bead, the formulation comprises a plurality of beads coated with the therapeutic agent which are then overcoated with a diffusion barrier coating.

The diffusion barrier coating comprises an anionic polymer and optionally other excipients. Examples of anionic polymers for use in the present invention include for example and without limitation, acrylic acid polymers and copolymers, methacrylic acid polymers and copolymers, non-acrylic enteric coating polymers, mixtures thereof, and the like. Also useful in accordance with the present invention in place of, or in addition to, the anionic polymer are cellulose derivatives (e.g., carboxymethylcellulose), starches (carboxymethyl starch), gums (xanthan gum), mixtures thereof, and the like, which have affinity for the protonated therapeutic agent included in the formulation.

Examples of acrylic acid polymers and copolymers, and methacrylic acid polymers and copolymers include, for example and without limitation, carboxypolymethylene, poly(acrylic acid), polyacrylamide, methyl methacrylate copolymers, ethoxyethyl methacrylates, cyanoethyl methacrylate, poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers.

In certain preferred embodiments, the acrylic polymer is comprised of one or more ammonio methacrylate copolymers. Ammonio methacrylate copolymers are well known in the art, and are described in NF XVII as fully polymerized copolymers of acrylic and methacrylic acid esters with a low content of quaternary ammonium groups.

Certain methacrylic acid ester-type polymers of a family of copolymers synthesized from diethylaminoethyl methacrylate and other neutral methacrylic esters, also known as methacrylic acid copolymer or polymeric methacrylates, commercially available as Eudragit^{®} from Röhm Tech, Inc may also be useful for purposes of the present invention. There are several different types of Eudragit^{®}. For example, Eudragit^{®} E is an example of a methacrylic acid copolymer which swells and dissolves in acidic media. Eudragit^{®} L is a methacrylic acid copolymer which does not swell at about pH < 5.7 and is soluble at about pH > 6. Eudragit^{®} S does not swell at about pH < 6.5 and is soluble at about pH > 7. Eudragit^{®} RL and Eudragit^{®} RS are water swellable, and the amount of water absorbed by these polymers is pH-dependent, however, dosage forms coated with Eudragit^{®} RL and RS are pH-independent.

The diffusion barrier coating may comprise a mixture of two acrylic resin lacquers commercially available from Rohm Pharma under the Tradenames Eudeagit^{®} RL30D and Eudragit^{®} RS30D, respectively. Eudragit^{®} RL30D and Eudragit^{®} RS30D are copolymers of acrylic and methacrylic esters with a low content of quaternary ammonium groups, the molar ratio of ammonium groups to the remaining neutral (meth)acrylic esters being 1:20 in Eudragit^{®} RL30D and 1:40 in Eudragit^{®} RS30D. The mean molecular weight is about 150,000.

Certain non-acrylic enteric coating polymers for use in the diffusion barrier coating of the present invention include, for example and without limitation, cellulose acetate phthalate, hydroxypropyl methylcellulose phthalate, carboxymethyl ethylcellulose, hydroxypropyl methylcellulose acetate succinate, polyvinyl acetate phthalate, cellulose acetate trimellatate, cellulose acetophthalate, cellulose acetate terephthalate, polyvinyl alcohol phthalate, mixtures thereof, and the like.

Other optional ingredients can be included in the diffusion barrier coating, such as for example, plasticizers, binders, lubricants, glidants, fillers, etc, described hereinafter. In certain preferred embodiments, the diffusion barrier coating includes a plasticizer as described hereinafter.

The diffusion barrier coating is applied onto the substrates comprising the therapeutic agent in an amount of from about 0.1 to about 20% by weight, preferably from about 1 to about 10 % by weight of the substrates comprising the therapeutic agent. As with the application of the therapeutic agent to the substrates, the diffusion barrier coating may be applied by spraying a suitable solution or dispersion comprising the anionic polymer employing a suitable mixture of solvents and using techniques known in the art. The diffusion barrier coating preferably prevents or decreases the amount of migration of the therapeutic agent from the dosage form by having an affinity for the protonated therapeutic agent of the substrate.

After the substrates comprising the therapeutic agent are coated with the diffusion barrier coating, they are then overcoated with a coating comprising a hydrophobic material. Preferably the hydrophobic material provides for the controlled release of the therapeutic agent, or the sequestration of the therapeutic agent.

Certain hydrophobic materials for inclusion in the coating include, for example and without limitation, cellulosic materials and polymers, acrylic polymers, mixtures thereof, and the like.

In certain embodiments the hydrophobic material comprises a cellulosic material or cellulosic polymers, including alkylcelluloses. Simply by way of example, one preferred alkylcellulosic polymer is ethylcellulose, although the artisan will appreciate that other cellulose and/or alkylcellulose polymers may be readily employed, singly or in any combination, as all or part of the hydrophobic coating according to the invention.

One commercially available aqueous dispersion of ethylcellulose is Aquacoat^{®} (FMC Corp., Philadelphia, Pennsylvania, U.S.A.). Aquacoat^{®} is prepared by dissolving the ethylcellulose in a water-immiscible organic solvent and then emulsifying the same in water in the presence of a surfactant and a stabilizer. After homogenization to generate submicron droplets, the organic solvent is evaporated under vacuum to form a pseudolatex. The plasticizer is not incorporated in the pseudolatex during the manufacturing phase. Thus, prior to using the same as a coating, it is necessary to intimately mix the Aquacoat^{®} with a suitable plasticizer prior to use.

Another aqueous dispersion of ethylcellulose is commercially available as Surelease^{®} (Colorcon, Inc., West Point, Pennsylvania, U.S.A.). This product is prepared by incorporating plasticizer into the dispersion during the manufacturing process. A hot melt of a polymer, plasticizer (dibutyl sebacate), and stabilizer (oleic acid) is prepared as a homogeneous mixture, which is then diluted with an alkaline solution to obtain an aqueous dispersion which can be applied directly onto substrates.

In certain embodiments the hydrophobic material comprises a pharmaceutically acceptable acrylic polymer as desired above, including but not limited to acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers.

In certain preferred embodiments, the acrylic polymer is comprised of one or more ammonio methacrylate copolymers. In order to obtain a desirable dissolution profile, it may be necessary to incorporate two or more ammonio methacrylate copolymers having differing physical properties, such as different molar ratios of the quaternary ammonium groups to the neutral (meth)acrylic esters.

Certain methacrylic acid ester-type polymers are useful for preparing pH-dependent coatings which may be used in accordance with the present invention. For example, there are a family of copolymers synthesized from diethylaminoethyl methacrylate and other neutral methacrylic esters, also known as methacrylic acid copolymer or polymeric methacrylates, commercially available as Eudragit^{®} from Röhm Tech, Inc. As described above, there are several different types of Eudragit^{®}.

In certain preferred embodiments, the acrylic coating comprises a mixture of two acrylic resin lacquers commercially available from Rohm Pharma under the Tradenames Eudragit^{®} RL30D and Eudragit^{®} RS30D as described above. The code designations RL (high permeability) and RS (low permeability) refer to the permeability properties of these agents. Eudragit^{®} RL/RS mixtures are insoluble in water and in digestive fluids. However, coatings formed from the same are swellable and permeable in aqueous solutions and digestive fluids.

The Eudragit^{®} RL/RS dispersions of the present invention may be mixed together in any desired ratio in order to ultimately obtain a controlled release formulation having a desirable dissolution profile. Desirable controlled release formulations may be obtained, for instance, from a retardant coating derived from 100% Eudragit^{®} RL, 50% Eudragit^{®} RL and 50% Eudragit^{®} RS, and 10% Eudragit^{®} RL: 90% Eudragit^{®} RS. Of course, one skilled in the art will recognize that other acrylic polymers may also be used, such as, for example, Eudragit^{®} L.

In certain embodiments, wherein the coating comprises an aqueous dispersion of a hydrophobic material such as for example an alkylcellulose or an acrylic polymer, the inclusion of an effective amount of a plasticizer in the aqueous dispersion of hydrophobic material will further improve the physical properties of the controlled release coating. For example, because ethylcellulose has a relatively high glass transition temperature and does not form flexible films under normal coating conditions, it is preferable to incorporate a plasticizer into an ethylcellulose coating containing controlled release coating before using the same as a coating material. Generally, the amount of plasticizer is included in a coating solution in an amount of from about 1 to about 50 percent by weight of the hydrophobic material. Concentration of the plasticizer, however, can only be properly determined after careful experimentation with the particular coating solution and method of application.

Examples of suitable plasticizers for ethylcellulose include, but are not limited to, water insoluble plasticizers such as dibutyl sebacate, diethyl phthalate, triethyl citrate, tributyl citrate, and triacetin, although it is possible that other water-insoluble plasticizers (such as acetylated monoglycerides, phthalate esters, castor oil, etc.) may be used.

Examples of suitable plasticizers for the acrylic polymers of the present invention include, but are not limited to citric acid esters such as triethyl citrate NF XVI, tributyl citrate, dibutyl phthalate, and possibly 1,2-propylene glycol. Other plasticizers which have proved to be suitable for enhancing the elasticity of the films formed from acrylic films such as Eudragit^{®} RL/RS lacquer solutions include polyethylene glycols, propylene glycol, diethyl phthalate, castor oil, and triacetin. Triethyl citrate is an especially preferred plasticizer for use in the present invention.

In certain embodiments where an aqueous dispersion of a hydrophobic polymer such as an alkylcellulose is applied to the substrate, the coated substrate is cured at a temperature above the glass transition temperature of the plasticized polymer and at a relative humidity above ambient conditions, until an endpoint is reached at which the coated formulation attains a dissolution profile which is substantially unaffected by exposure to storage conditions, e.g., of elevated temperature and/or humidity. Generally, in such formulations the curing time is about 24 hours or more, and the curing conditions may be, for example, about 60° C and 85% relative humidity. Detailed information concerning the stabilization of such formulations is set forth in U.S. Patent Nos. 5,273,760; 5,681,585; and 5,472,712.

In formulations where a controlled release coating comprising an aqueous dispersion of an acrylic polymer is applied to the substrate, it is preferred that the controlled release coated substrate is cured at a temperature above the glass transition temperature of the plasticized polymer until an endpoint is reached at which the controlled release coated formulation attains a dissolution profile which is substantially unaffected by exposure to storage conditions, e.g., of elevated temperature and/or humidity. Generally, the curing time is about 24 hours or more, and the curing temperature may be, for example, about 45° C. Detailed information concerning the stabilization of such formulations is set forth in U.S. Patent Nos. 5,286,493; 5,580,578; and 5,639,476.

The controlled release profile of the coated formulations of the invention can be altered, for example, by varying the amount of overcoating with the aqueous dispersion of hydrophobic material, altering the manner in which the plasticizer is added to the aqueous dispersion of hydrophobic material, by varying the amount of plasticizer relative to hydrophobic material, by the inclusion of additional ingredients or excipients, by altering the method of manufacture, combinations thereof, and the like.

The coating solutions of the present invention preferably contain, in addition to the plasticizer and solvent system (e.g., water), a colorant to provide elegance and product distinction. Color may be added, for example, to the aqueous dispersion of hydrophobic material. For example, color may be added to Aquacoat via the use of alcohol or propylene glycol based color dispersions, milled aluminum lakes and opacifiers such as titanium dioxide by adding color with shear to the water soluble polymer solution and then using low shear to the plasticized Aquacoat. Alternatively, any suitable method of providing color to the formulations of the present invention may be used. Suitable ingredients for providing color to the formulation when an aqueous dispersion of an acrylic polymer is used include titanium dioxide and color pigments, such as iron oxide pigments. The incorporation of pigments, may, however, increase the retardant effect of the controlled release coating.

The plasticized aqueous dispersion (e.g., solution or suspension) ofhydrophobic material may be applied onto the substrate comprising the therapeutic agent by spraying using any suitable spray equipment known in the art. The dispersion may be applied to the diffusion barrier coated substrates comprising the therapeutic agent in a conventional coating pan or, alternatively, using an automated system such as a CF granulator, for example a FREUND CF granulator, a GLATT fluidized bed processor, an AEROMATIC, a modified ACCELA-COTA or any other suitably automated bead coating equipment.

Preferably 2-25 ml of the solution/suspension is applied per coat per kilogram of substrate. In an automated system the total amount of solution/suspension applied to the substrate is the same as that applied in a conventional coating pan, except that the solution/suspension is applied continuously.

Preferably, when a coating pan is used the coating is applied at a rate of 20-30 coats between each drying step until all of the coats have been applied. Between applications the substrates may be dried for more than 12 hours at a temperature of 50°C = 60°C., most suitably 55°C.

In an automated coating system the rate of application of solution/suspension may be 0.5-10 g/kg of substrate/min.

In a preferred method, a Wurster fluidized-bed system is used in which an air jet, injected from underneath, fluidizes the substrate and effects drying while the acrylic polymer coating is sprayed on. In certain embodiments, a sufficient amount of the aqueous dispersion of hydrophobic material is applied to the diffusion barrier coated substrate comprising the therapeutic agent to obtain a predetermined controlled release of the therapeutic agent (i.e., drug) when the coated substrate is exposed to aqueous solutions, e.g., gastric fluid.

In certain embodiments a further overcoat of a film-former, such as Opadry^{®}, is optionally applied to the substrates after coating the substrates with the hydrophobic coating. This overcoat is provided, if at all, preferably in order to substantially reduce agglomeration of the beads.

After the diffusion barrier coated substrates (e.g., beads) are overcoated with the hydrophobic coating, the overcoated substrates (e.g., controlled release beads) which are formed may be filled into hard or soft gelatin capsules. Alternatively, the overcoated substrates may be compressed into tablets using a binder and/or hardening agent commonly employed in tabletting such as, for example and without limitation, microcrystalline cellulose sold under the Trade Mark "AVICEL" or a co-crystallised powder of highly modified dextrins (3% by weight) and sucrose sold under the Trade Mark "DI-PAC" in such a way that the specific dissolution rate of the controlled release substrates (e.g., beads) is maintained.

Following the formation of the mixture into a tablet, it may be desirable to apply a very thin coating to the external surface of the tablet. The function of the coating, when applied, is to enhance the intactness of the tablet. The coating may comprise a polymer, such as polyvinyl alcohol or a polyvinylpyrrolidol, which, maintains the tablet intact but does not inhibit the capillary uptake by the tablet once placed in the aqueous environment of use (e.g., gastrointestinal system), although dissolution time may be slightly increased when a coating is applied to the tablet.

In certain embodiments, the formulations of the present invention may further include a lubricant which may be mixed with any of the coatings prior to application. Suitable lubricants include for example talc, magnesium stearate, sodium stearate, stearic acid, calcium stearate, magnesium oleate, oleic acid, potassium oleate, caprylic acid, sodium stearyl fumarate, and magnesium palmitate, mixtures thereof and the like. Generally, when a lubricant is present, the quantity of lubricant will be from about 0.1% to about 10%, preferably about 0.1% to about 5%.

In certain embodiments, the formulations of the present invention may further include a binder. The binder may be any pharmaceutically acceptable binder known to those skilled in the art. Such binders include, for example, polyvinylpyrrolidone, natural and synthetic gums including gum arabic, hydroxypropylmethylcellulose, hydroxypropylcellulose, carboxymethylcellulose, methylcellulose, pullulan, dextrin, starch, mixtures thereof and the like. The binder may be mixed with any of the coatings prior to application or may be dissolved or dispersed in an aqueous or organic solution, or a mixture thereof Aqueous binder solutions or dispersions are especially preferred. Suitable binding agents which are generally considered to be water-soluble include polyvinylpyrrolidone, hydroxypropylmethylcellulose, and maize starch. Many other water-soluble binding agents which would be suitable for use in conjunction with the present invention are known to those skilled in the art.

In certain embodiments, the compositions of the present invention further comprise a pharmaceutically acceptable carrier. Generally, the carriers to be used herein are, for example and without limitation, microcrystalline cellulose, polyvinylpyrrolidone, lactose, dextrose, sucrose, starch, sorbitol, mannitol, mixtures thereof, and the like. Other examples of pharmaceutically acceptable carriers and excipients that may be used to formulate oral dosage forms are described in the Handbook of Pharmaceutical Excipients, American Pharmaceutical Association (1986).

Other optional ingredients that can be included in the formulations of the present invention include glidants such as talc, titanium dioxide, magnesium stearate, silicon dioxide, dibutyl sebacate, ammonium hydroxide, oleic acid colloidal silica, mixtures thereof and the like, which may be mixed with any of the coatings prior to application, and/or dissolved or dispersed in an aqueous and/or organic solvent prior to application.

In certain embodiments, the formulations of the present invention further include a release=modifying agent. The release=modifying agents which function as pore=formers may be organic or inorganic, and include materials that can be dissolved, extracted or leached from the controlled release coating in the environment of use. The pore-formers may comprise one or more hydrophilic materials such as hydroxypropylmethylcellulose. In certain preferred embodiments, the release-modifying agent is selected from hydroxypropylmethylcellulose, lactose, metal stearates, and mixtures of any of the foregoing.

The controlled release coatings of the present invention can also include erosion-promoting agents such as starch and gums.

The controlled release coatings of the present invention can also include materials useful for making microporous lamina in the environment of use, such as polycarbonates comprised of linear polyesters of carbonic acid in which carbonate groups reoccur in the polymer chain.

The controlled release coatings of the present invention may also include an exit means comprising at least one passageway, orifice, or the like. The passageway may be formed by such methods as those disclosed in U.S. Patent Nos. 3,845,770; 3,916,889; 4,063,064; and 4,088,864. The passageway can have any shape such as round, triangular, square, elliptical, irregular, etc.

In certain embodiments it may be necessary to include a stabilizer in the formulation of the present invention to prevent the degradation of the therapeutic agent. For example, a degradation product of naltrexone hydrochloride includes for example and without limitation, 10-hydroxynaltrexone; 10-ketonaltrexone; 2,2' bisnaltrexone (pseudonaltrexone); oxides of 2,2' bisnaltrexone; dioxides of 2,2' bisnaltrexone; aldol adduct of naltrexone and 10-hydroxynaltrexone; aldol adduct of naltrexone and 10-ketonaltrexone; naltrexone-N-oxide; 10-hydroxynaltrexone-N-oxide; 10-ketonaltrexone-N-oxide; semiquinones of naltrexone; free radical peroxides of naltrexone; aldol adduct of naltrexone; aldol adducts of naltrexone coupled at the 7,6 position; aldol adducts of naltrexone coupled at the 6,5 position; ether-linked adduct of naltrexone; ether-linked adduct of naltrexone and 10-hydroxynaltrexone; ether-linked adduct of naltrexone and 10-ketonaltrexone; dehydrogenated naltrexone; hydroxy-naltrexone; keto-naltrexone; salts thereof and mixtures thereof; and the like.

Stabilizers of use in this invention for preventing the degradation of, e.g., naltrexone hydrochloride, include for example and without limitation, organic acids, carboxylic acids, acid salts of amino acids (e.g., cysteine, L-cysteine, cysteine hydrochloride, glycine hydrochloride or cystine dihydrochloride), sodium metabisulphite, ascorbic acid and its derivatives, malic acid, isoascorbic acid, citric acid, tartaric acid, palmitic acid, sodium carbonate, sodium hydrogen carbonate, calcium carbonate, calcium hydrogen phosphate, sulphur dioxide, sodium sulphite, sodium bisulphate, tocopherol, as well as its water- and fat-soluble derivatives, such as e.g., tocofersolan or tocopherol acetate, sulphites, bisulphites and hydrogen sulphites or alkali metal, alkaline earth metal and other metals, PHB esters, gallates, butylated hydroxyanisol (BHA) or butylated hydroxytoluene (BHT), and 2,6-di-t-butyl-.alpha.-dimethylamino-p-cresol, t-butylhydroquinone, di-t-amylhydroquinone, di-t-butylhydroquinone, butylhydroxytoluene, butylhydroxyanisole, pyrocatechol, pyrogallol, propyl/gallate, and nordihydroguaiaretic acid, as well as lower fatty acids, fruit acids, phosphoric acids, sorbic and benzoic acids as well as their salts, esters, derivatives and isomeric compounds, ascorbyl palmitate, lecithins, mono- and polyhydroxylated benzene derivatives, ethylenediamine-tetraacetic acid and its salts, citraconic acid, conidendrine, diethyl carbonate, methylenedioxyphenols, kephalines, β,β'-dithiopropionic acid, biphenyl and other phenyl derivatives, pharmaceutically acceptable salts thereof, and mixtures thereof. In certain preferred embodiments, the stabilizer is BHT. In other preferred embodiments, the stabilizer is ascorbic acid. All or part of the ascorbic acid can be replaced with a metal or ammonium ascorbate, e.g., sodium, potassium and/or iodine ascorbate(s). Sodium ascorbate is preferred.

In addition to the above ingredients, the compositions of the present invention may also contain suitable quantities of other materials, e.g., granulating aids, colorants, and flavorants that are conventional in the pharmaceutical art. The quantities of these additional materials will be sufficient to provide the desired effect to the desired composition.

In certain embodiments of the present invention, the therapeutic agent may also be included in immediate release coating in the formulation. The immediate release coating of the therapeutic agent is included in an amount which is effective to reduce the time to maximum concentration of the therapeutic agent in the blood (e.g., plasma). In certain embodiments, the immediate release layer is coated over the controlled release coating. On the other hand, the immediate release layer may be coated over the surface of tablets or capsules of the final formulation. One skilled in the art would recognize still other alternative manners of incorporating an immediate release form of the therapeutic agent into the formulation. In certain alternate embodiments an immediate release layer comprising a different therapeutic agent other than the therapeutic agent of the controlled release substrate may be coated over the control release coating comprising the hydrophobic polymer.

The present invention is further directed to a process for the preparation of the oral pharmaceutical formulations described herein, and as defined in claim 14.

In a preferred embodiment the therapeutic agent is applied onto the substrate. Thereafter, the diffusion barrier coating is applied on said substrate over the therapeutic agent. Preferably the diffusion barrier coating is applied until a weight gain, ranging from about 0.1 to 30%, preferably from about 1 to 20%, is reached. Then, the hydrophobic coating is overcoated on the diffusion barrier coating.

The coatings, including the coating with the therapeutic agent, are preferably applied by means of a film coating process, either in a fluid bed apparatus or in a pan coat, or a atomization process, or alternatively a press coating process.

In certain preferred embodiments, the coating layers are applied on the substrate by means of a film coating process, by spraying an aqueous polymeric dispersion or an organic or hydro-organic solvent polymeric dispersion with a solid content ranging between 1 and 50% w/w, preferably ranging between 1 and 25%.

Spheroids comprising the therapeutic agent may also be prepared, for example, by adding a spheronizing agent to the substrate compositions described above prior to or after coating the substrates with the controlled release coating.

The formulations of the present invention comprise a therapeutically effect amount of the therapeutic agent. In certain preferred embodiments of the present invention, the formulations comprise a plurality of the resultant controlled release substrates to provide a therapeutically effective amount of the therapeutic agent. In certain preferred embodiments, the therapeutic agent is in an amount sufficient to provide an effective controlled release dose when ingested and contacted by an environmental fluid, e.g., gastric fluid or dissolution media.

The final form of the pharmaceutical preparations made in accordance with the invention can vary greatly. Thus, tablets, caplets, capsules, sachets, and the like are contemplated. Tablets, caplets, and capsules are preferred.

The present invention will now be more fully described with reference to the accompanying examples.

### EXAMPLE 1 (comparative)

In Example 1, naltrexone HCl beads were prepared having the composition listed in Table 1:

**TABLE 1**

| | Ingredients | Amt/unit (mg) | Amt/batch (g) |
|---|---|---|---|
| Step 1. Drug layering | Naltrexone HCl anhydrous | 0.658 | 12.15 |
| | Non-pareil beads (30/35 mesh) | 79.788 | 1473.0 |
| | Opadry Clear (Hydroxypropymethyl cellulose) | 0.775 | 14.73 |
| Step 2. Anionic polymer coat | Eudragit L30D (dry) | 3.023 | 55.8 |
| | Triethyl Citrate | 0.756 | 13.95 |
| | Glyceryl Monostearate | 0.284 | 5.25 |
| Step 3. Controlled release coat | Eudragit RS30D (dry) | 32.5 | 600.0 |
| | Triethyl citrate | 6.5 | 120.0 |
| | Cab-o-sil | 1.625 | 30.0 |
| Step 4. Seal coat | Opadry Clear (Hydroxypropylmethyl cellulose) | 4.062 | 75.0 |
| Total (on dry basis) | | 130 | 2400 |

### Bead Manufacturing Process

1. Dissolve naltrexone HCl and Opadry Clear in water. Spray the drug solution onto non-pareil beads in a fluid bed coater with Wurster insert.
2. Disperse Eudragit L30D, Triethyl citrate, and glyceryl monostearate in water. Spray the dispersion onto the drug-loaded beads in the fluid bed coater.
3. Disperse Eudragit RS30D, triethyl citrate, and cabosil in water. Spray the dispersion onto the beads in the fluid bed coater.
4. Dissolve Opadry Clear in water. Spray the solution onto the beads in the fluid bed
5. Cure the beads at 40°C for 24 hours.

### EXAMPLE 2 (comparative)

In Example 2, Naltrexone HCl beads were prepared as in Example 1 (BHT was added (dissolved) in step 1), having the composition listed in Table 2 below:

**TABLE 2**

| | Ingredients | Amt/unit (mg) | Amt/batch (g) |
|---|---|---|---|
| Step 1. Drug layering | Naltrexone HCl anhydrous | 0.658 | 12.15 |
| Non-pareil | beads (30/35 mesh) | 79.788 | 1473.0 |
| | Opadry Clear (Hydroxypropymethyl cellulose) | 0.775 | 14.31 |
| | BHT | 0.029 | 0.54 |
| Step 2. Anionic polymer coat | Eudragit L30D (dry) | 3.023 | 55.8 |
| | Triethyl Citrate | 0.756 | 13.95 |
| | Glyceryl Monostearate | 0.284 | 5.25 |
| Step 3, Controlled release coat | Eudragit RS30D (dry) | 32,5 | 600,0 |
| | Triethyl citrate | 6.5 | 120.0 |
| | Cabosil | 1.625 | 30.0 |
| Step 4. Seal coat | Opadry Clear (Hydroxypropylmethyl cellulose) | 4.062 | 75.0 |
| Total (on dry basis) | | 130.0 | 2400.0 |

### EXAMPLE 3 (comparative)

In Example 3, Naltrexone HCl beads were prepared as in Example 1 (ascorbic acid was added (dissolved) in Step 1), having the composition listed in Table 3:

**TABLE 3**

| | Ingredients | Amt/unit (mg) |
|---|---|---|
| Step 1. Drug layering | Naltrexone HCl anhydrous | 0.584 |
| | Non-pareil beads (30/35 mesh) | 80.26 |
| | Opadry Clear (Hydroxypropymethyl cellulose) | 0.341 |
| | Ascorbic acid | 0.065 |
| Step 2. Anionic polymer coat | Eudragit L30D (dry) | 3.023 |
| | Triethyl Citrate | 0.756 |
| | Glyceryl Monostearate | 0.284 |
| Step 3. Controlled release coat | Eudragit RS30D (dry) | 32,5 |
| | Triethyl citrate | 6.5 |
| | Cabosil | 1.625 |
| Step 4. Seal coat | Opadry Clear (Hydroxypropylmethyl cellulose) | 3.532 |
| | Cab-o-sil | 0.531 |
| Total (on dry basis) | | 130.0 |

### EXAMPLE 4

In Example 4, Naltrexone HCl beads were prepared as in Example 1 (ascorbic acid and sodium ascorbate were added (dissolved) in step 1), having the composition listed in Table 4 below:

**TABLE 4**

| | Ingredients | Amt/unit (mg) |
|---|---|---|
| Step 1. Drug coating | Naltrexone HCl anhydrous | 2.00 |
| | Non-pareil beads (30/35 mesh) | 39.08 |
| | Opadry Clear (Hydroxypropymethyl cellulose) | 2.00 |
| | Sodium ascorbate | 0.067 |
| | Ascorbic acid | 0.133 |
| Step 2. Diffusion barrier coat | Eudragit L 55 | 2.164 |
| | Triethyl Citrate | 0.433 |
| | Cab-O-Sil | 0.108 |
| Step 3. Controlled release coat | Eudragit RS | 17.475 |
| | Triethyl citrate | 3.495 |
| | Cab-O-Sil | 0.874 |
| Step 4. Seal coat | Opadry Clear (Hydroxypropylmethyl cellulose) | 1.899 |
| | Cab-O-Sil | 0.271 |
| Total | | 69.998 |

### EXAMPLE 5

In Example 5, a formulation was prepared as in Example 4 (glycerol monostearate was used in place of cabosil), having the formulation listed in Table 5 below:

**TABLE 5**

| | Ingredients | Amt/unit (mg) |
|---|---|---|
| Step 1. Drug coating | Naltrexone HCl anhydrous | 2.00 |
| | Non-pareil beads (30/35 mesh) | 38.98 |
| | Opadry Clear (Hydroxypropymethyl cellulose) | 2.00 |
| | Sodium ascorbate | 0.067 |
| | Ascorbic acid | 0.133 |
| Step 2. Diffusion barrier coat | Eudragit L 55 | 2.159 |
| | Triethyl Citrate | 0.432 |
| | Glyceryl monostearate | 0.216 |
| Step 3. Controlled release coat | Eudragit RS | 17.475 |
| | Triethyl citrate | 3.495 |
| | Cab-O-Sil | 0.874 |
| Step 4. Seal coat | Opadry Clear (Hydroxypropylmethyl cellulose) | 1.899 |
| | Cab-O-Sil | 0.271 |
| Total | | 70.001 |

### Example 6

Oxycodone controlled release beads are prepared according to the following formula and process:

**Table 6**

| **Formula Oxycodone HCl beads** | | |
|---|---|---|
| | Ingredients | Amt/unit* (mg) |
| Step 1. Drug layering | Oxycodone HCl | 10.5 |
| | Non-pareil beads (30/35 mesh) | 45.349 |
| | Opadry Clear | 2.5 |
| Step 2. Controlled release coat | Eudragit RS30D (dry) | 7.206 |
| | Eudragit RL30D (dry) | 0.379 |
| | Triethyl citrate | 1.517 |
| | Cabosil | 0.379 |
| Step 3. Seal coat | Opadry Clear (Hydroxypropylmethyl cellulose) | 1.899 |
| | Cabosil | 0.271 |
| Total | | 70.0 |

### Bead Manufacturing Procedure

1. Dissolve oxycodone HCl and Opadry (HPMC) in water. Spray the drug solution onto non-pareil beads in a fluid bed coater with Wurster insert.
2. Disperse Eudragit RS, Eudragit RL, triethyl citrate, and Cabosil in water. Spray the dispersion onto the beads in the fluid bed coater.
3. Dissolve Opadry in water. Spray the solution onto the beads in the fluid bed coater.
4. Cure the beads at 45°C for 24 hours.

### Example 7

Oxycodone controlled release beads with an anionic polymer coating are prepared according to the following formula and process:

**Table 7**

| **Formula Oxycodone HCl beads** | | |
|---|---|---|
| | Ingredients | Amt/unit* (mg) |
| Step 1. Drug layering | Oxycodone HCl | 10.5 |
| | Non-pareil beads (30/35 mesh) | 45.349 |
| | Opadry Clear | 2.5 |
| Step 2. Anionic polymer coating | Eudragit L30D (dry) | 2.0 |
| | Triethyl citrate | 0.4 |
| | Carbosil | 0.1 |
| Step 3. Controlled release coat | Eudragit RS30D (dry) | 7.206 |
| | Eudragit RL30D (dry) | 0.379 |
| | Triethyl citrate | 1.517 |
| | Cabosil | 0.379 |
| Step 4. Seal coat | Opadry Clear (Hydroxypropylmethyl cellulose) | 1.899 |
| | Cabosil | 0.271 |
| Total | | 72.5 |

### Bead Manufacturing Procedure

1. Dissolve oxycodone HCl and Opadry (HPMC) in water. Spray the drug solution onto non-pareil beads in a fluid bed coater with Wurster insert.
2. Disperse Eudragit L30D, triethyl citrate, and Cabosil in water. Spray the dispersion onto the beads in the fluid bed coater.
3. Disperse Eudragit RS, Eudragit RL, triethyl citrate, and Cabosil in water. Spray the dispersion onto the beads in the fluid bed coater.
4. Dissolve Opadry in water. Spray the solution onto the beads in the fluid bed coater.
5. Cure the beads at 45°C for 24 hours.

It would be expected that the dissolution of Example 7 would be slower than the dissolution of Example 6 due to the inclusion of the anionic polymer coating.

### Example 8 (comparative)

In Example 8, naltrexone beads without a diffusion barrier coat were prepared having the composition listed in Table 8 below.

**Table 8**

| | Ingredients | Amt/unit (mg) | Amt/batch (g) |
|---|---|---|---|
| Step1. Drug Layering | Naltrexone HCl anhydrous | 1.000 | 14.00 |
| | Non-pareil beads (30/33 mesh) | 47.998 | 672.00 |
| | Plasdone C-30 (Povidone) | 0.500 | 7.00 |
| | Talc, USP | 0.500 | 7.00 |
| Step 2. Seal coat | Opadry Clear (Hydroxypropylmethyl cellulose) | 2.500 | 35.00 |
| Step 3. Sustained release coat | Eudradit RS30D (dry) | 8.814 | 123.40 |
| | Dibutyl Sebacate | 1.764 | 24.70 |
| | Talc, USP | 4.407 | 61.70 |
| | Tween 80 | 0.018 | 0.25 |
| Step 4. Seal coat | Opadry Clear (Hydroxypropylmethyl cellulose) | 2.500 | 35.00 |
| Total (on dry basis) | | 70.001 | 980.05 |

### Bead Manufacturing Process

1. Dissolve naltrexone HCl and Plasdone in water. Disperse the talc in the drug solution. Spray the drug dispersion onto the non-pareil beads in the fluid bed coater with Wurster insert.
2. Dissolve Opadry clear in water. Spray the solution onto the drug loaded beads in the fluid bed coater.
3. Disperse the Eudragit RS30D, Dibutyl Sebacate, Tween 80 and Talc in water. Spray the dispersion onto beads in the fluid bed coater.
4. Dissolve Opadry clear in water. Spray the solution onto beads in the fluid bed coater.

### Example 9

In Example 9, the formulations from Example 8 and Examples 1-5 were dissolution tested using the dissolution method below.

### Dissolution Method

1. Apparatus- USP Type II (paddle), 50 rpm at 37°C.
2. Sampling time- 1, 2, 4, 12, 24, and 36 hours (1, 2, 4, 8, and 18 hours for Example 8).
3. Media- 900 ml pH 6.5 phosphate buffer.
4. Analytical method- High performance liquid chromatography.
Dissolution Results for Example 8 are listed in Table 9 below:

**Table 9**

| Time (hrs.) | % Dissolved |
|---|---|
| 1 | 2.0 |
| 2 | 22.0 |
| 4 | 43.0 |
| 8 | 59.0 |
| 18 | 74.0 |

Dissolution results for Examples 1-5 are listed in Table 10 below

**Table 10**

| | **Example 1** | **Example 2** | **Example 3** | **Example 4** | **Example 5** |
|---|---|---|---|---|---|
| Time (hrs.) | % Dissolved | % Dissolved | % Dissolved | % Dissolved | % Dissolved |
| 1 | 0.0 | 0.9 | 0.0 | 0.4 | 0.4 |
| 2 | 0.2 | 4.7 | 0.0 | 0.6 | 0.6 |
| 4 | 0.1 | 5.1 | 0.0 | 0.7 | 0.8 |
| 8 | 0.4 | 5.8 | 0.0 | 0.8 | 1.0 |
| 12 | 0.6 | 8.0 | 0.2 | 1.0 | 1.2 |
| 24 | 1.0 | 15.2 | 0.5 | 1.4 | 1.5 |
| 36 | 2.3 | 19.1 | 1.2 | 2.2 | 2.8 |

## Claims

1. A pharmaceutical formulation comprising:
a substrate comprising an opioid antagonist, wherein the opioid antagonist is protonated;
a diffusion barrier coating comprising an anionic polymer coated over said substrate; and
a coating comprising a hydrophobic material coated over said diffusion barrier coating, wherein the diffusion barrier coating is applied in an amount of from 0.1 to 20% by weight of the substrate comprising said opioid antagonist
with the exception of the following formulations:
A) Non-pareil beads (30/35 mesh) (39.98 mg), first coated with naltrexone HCl (2.1 mg), hydroxypropylmethyl cellulose (Opadry Clear^{®}) (0.4 mg), sodium ascorbate (0.027 mg), and ascorbic acid (0.05 mg), then coated with a methacrylic acid - ethyl acrylate copolymer (1:1) (Eudragit L30D^{®}) (2.164 mg), triethyl citrate (0.433 mg), and fumed silica (Cabosil^{®}) (0.108 mg), then coated with ammonio methacrylate copolymer Type B (Eudragit RS30D^{®}) (17.475 mg), triethyl citrate (3.495 mg), and fumed silica (Cabosil^{®}) (0.874 mg), and finally coated with hydroxypropylmethyl cellulose (Opadry Clear^{®}) (1.899 mg) and fumed silica (0.271 mg);
B) Non-pareil beads (30/35 mesh) (67.264 mg), first coated with naltrexone HCl (0.609 mg), and hydroxypropylmethyl cellulose (Opadry Clear^{®}) (0.547 mg), then coated with a methacrylic acid - ethyl acrylate copolymer (1:1) (Eudragit L30D^{®}) (2.545 mg), triethyl citrate (0.636 mg), and glyceryl monostearate (0.239 mg), then coated with ammonio methacrylate copolymer Type B (Eudragit RS30D^{®}) (43.789 mg), triethyl citrate (8.758 mg), and fumed silica (Cabosil^{®}) (2.189 mg), and finally coated with hydroxypropylmethyl cellulose (Opadry Clear^{®}) (2.053 mg) and fumed silica (1.368 mg);
C) Non-pareil beads (30/35 mesh) (79.788 mg), first coated with naltrexone HCl (0.658 mg), and hydroxypropylmethyl cellulose (Opadry Clear^{®}) (0.775 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (4.062 mg);
D) Non-pareil beads (30/35 mesh) (79.788 mg), first coated with naltrexone HCl (0.658 mg), hydroxypropylmethyl cellulose (Opadry Clear^{®}) (0.775 mg), and butylated hydroxytoluene (BHT) (0.029 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (4.062 mg);
E) Non-pareil beads (30/35 mesh) (80.179 mg), first coated with naltrexone HCl (0.584 mg), hydroxypropylmethyl cellulose (Opadry Clear^{®}) (0.341 mg), sodium ascorbate (0.065 mg), and EDTA (0.065 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (4.062 mg), and fumed silica (Cabosil^{®}) (1.625 mg);
F) Non-pareil beads (30/35 mesh) (80.26 mg), first coated with naltrexone HCl (0.584 mg), hydroxypropylmethyl cellulose (Opadry Clear^{®}) (0.341 mg), and ascorbic acid (0.065 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (3.532 mg), and fumed silica (Cabosil^{®}) (0.531 mg); and
G) Non-pareil beads (30/35 mesh) (80.211 mg), first coated with naltrexone HCl (0.61 mg), hydroxypropylmethyl cellulose (Opadry Clear^{®}) (0.919 mg), propyl gallate (0.00581 mg), and EDTA (0.00349 mg), then coated with a methacrylic acid - ethyl acrylate copolymer (1:1) (Eudragit L30D^{®}) (3.022 mg), triethyl citrate (0.755 mg), and glyceryl monostearate (0.29 mg), then coated with ammonio methacrylate copolymer Type B (Eudragit RS30D^{®}) (32.534 mg), triethyl citrate (6.507 mg), and fumed silica (Cabosil^{®}) (1.627 mg), and finally coated with hydroxypropylmethyl cellulose (Opadry Clear^{®}) (3.538 mg), and fumed silica (Cabosil^{®}) (0.529 mg).

2. The pharmaceutical formulation of claim 1, wherein the substrate comprises the opioid antagonist coated over a core.

3. The pharmaceutical formulation of claim 2, wherein the core is a pharmaceutically acceptable inert bead.

4. The pharmaceutical formulation of claim 1, wherein the antagonist is dispersed in matrix multiparticulates.

5. The pharmaceutical formulation of claim 1, wherein the diffusion barrier coating is in an amount from about 0.1 to about 10 percent by weight of the substrate.

6. The pharmaceutical formulation of claim 1, wherein the opioid antagonist is in a therapeutically effective amount.

7. The pharmaceutical formulation of claim 1, comprising a plurality of said substrates.

8. The pharmaceutical formulation of claim 7, wherein said plurality of said substrates comprises a therapeutically effective amount of said opioid antagonist.

9. The pharmaceutical formulation of claim 1, wherein the coating comprising the hydrophobic material provides for the controlled release of the opioid antagonist.

10. The pharmaceutical formulation of claim 1, wherein the coating comprising the hydrophobic material provides for the sequestration of the opioid antagonist.

11. The pharmaceutical formulation of claim 1, wherein the hydrophobic material is selected from the group consisting of a cellulosic material, a cellulosic polymer, an acrylic polymer or copolymer, a methacrylic polymer or copolymer, and mixtures thereof.

12. The pharmaceutical formulation of claim 1, wherein said opioid antagonist is selected from the group consisting of naltrexone, naloxone and pharmaceutically acceptable salts thereof.

13. The pharmaceutical formulation of claim 1, wherein the anionic polymer is a non-acrylic enteric coating material, selected from the group consisting of cellulose acetate phthalate, hydroxypropyl methylcellulose phthalate, carboxymethyl ethylcellulose, hydroxypropyl methylcellulose acetate succinate, polyvinyl acetate phthalate, cellulose acetate trimellatate, cellulose acetophthalate, cellulose acetate terephthalate, polyvinyl alcohol phthalate, and mixtures thereof.

14. A process for preparing a pharmaceutical formulation comprising:
a) forming a substrate comprising an opioid antagonist, wherein the opioid antagonist is protonated;
b) applying a diffusion barrier coating comprising an anionic polymer onto said substrate; and
c) applying a coating comprising a hydrophobic material over said diffusion barrier coating, wherein the diffusion barrier coating is applied in an amount of from 0.1 to 20% by weight of the substrate comprising said opioid antagonist
with the exception of a process for preparing one of the following formulations:
A) Non-pareil beads (30/35 mesh) (39.98 mg), first coated with naltrexone HCl (2.1 mg), hydroxypropylmethyl cellulose (Opadry Clear^{®}) (0.4 mg), sodium ascorbate (0.027 mg), and ascorbic acid (0.05 mg), then coated with a methacrylic acid - ethyl acrylate copolymer (1:1) (Eudragit L30D^{®}) (2.164 mg), triethyl citrate (0.433 mg), and fumed silica (Cabosil^{®}) (0.108 mg), then coated with ammonio methacrylate copolymer Type B (Eudragit RS30D^{®}) (17.475 mg), triethyl citrate (3.495 mg), and fumed silica (Cabosil^{®}) (0.874 mg), and finally coated with hydroxypropylmethyl cellulose (Opadry Clear^{®}) (1.899 mg) and fumed silica (0.271 mg);
B) Non-pareil beads (30/35 mesh) (67.264 mg), first coated with naltrexone HCl (0.609 mg), and hydroxypropylmethyl cellulose (Opadry Clear^{®}) (0.547 mg), then coated with a methacrylic acid - ethyl acrylate copolymer (1:1) (Eudragit L30D^{®}) (2.545 mg), triethyl citrate (0.636 mg), and glyceryl monostearate (0.239 mg), then coated with ammonio methacrylate copolymer Type B (Eudragit RS30D^{®}) (43.789 mg), triethyl citrate (8.758 mg), and fumed silica (Cabosil^{®}) (2.189 mg), and finally coated with hydroxypropylmethyl cellulose (Opadry Clear^{®}) (2.053 mg) and fumed silica (1.368 mg);
C) Non-pareil beads (30/35 mesh) (79.788 mg), first coated with naltrexone HCl (0.658 mg), and hydroxypropylmethyl cellulose (Opadry Clear^{®}) (0.775 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (4.062 mg);
D) Non-pareil beads (30/35 mesh) (79.788 mg), first coated with naltrexone HCl (0.658 mg), hydroxypropylmethyl cellulose (Opadry Clear^{®}) (0.775 mg), and butylated hydroxytoluene (BHT) (0.029 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (4.062 mg);
E) Non-pareil beads (30/35 mesh) (80.179 mg), first coated with naltrexone HCl (0.584 mg), hydroxypropylmethyl cellulose (Opadry Clear^{®}) (0.341 mg), sodium ascorbate (0.065 mg), and EDTA (0.065 mg), then coated with a methacrylic acid - ethyl acrylate copolymer (1:1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (4.062 mg), and fumed silica (Cabosil^{®}) (1.625 mg);
F) Non-pareil beads (30/35 mesh) (80.26 mg), first coated with naltrexone HCl (0.584 mg), hydroxypropylmethyl cellulose (Opadry Clear^{®}) (0.341 mg), and ascorbic acid (0.065 mg), then coated with a methacrylic acid - ethyl acrylate copolymer (1: 1) (Eudragit L30D^{®}) (3.023 mg), triethyl citrate (0.756 mg), and glyceryl monostearate (0.284 mg), then coated with ammonio methacrylate copolymer Type B (Eudragit RS30D^{®}) (32.5 mg), triethyl citrate (6.5 mg), and fumed silica (Cabosil^{®}) (1.625 mg), and finally coated with hydroxypropylmethyl cellulose (Opadry Clear^{®}) (3.532 mg), and fumed silica (Cabosil^{®}) (0.531 mg); and
G) Non-pareil beads (30/35 mesh) (80.211 mg), first coated with naltrexone HCl (0.61 mg), hydroxypropylmethyl cellulose (Opadry Clear^{®}) (0.919 mg), propyl gallate (0.00581 mg), and EDTA (0.00349 mg), then coated with a methacrylic acid - ethyl acrylate copolymer (1:1) (Eudragit L30D^{®}) (3.022 mg), triethyl citrate (0.755 mg), and glyceryl monostearate (0.29 mg), then coated with ammonio methacrylate copolymer Type B (Eudragit RS30D^{®}) (32.534 mg), triethyl citrate (6.507 mg), and fumed silica (Cabosil^{®}) (1.627 mg), and finally coated with hydroxypropylmethyl cellulose (Opadry Clear^{®}) (3.538 mg), and fumed silica (Cabosil^{®}) (0.529 mg).

15. The process of claim 14, wherein said opioid antagonist is applied onto said substrate.

16. The process of claim 14, wherein the substrate is a pharmaceutically acceptable inert bead.

17. The process of claim 14, wherein the substrate is a matrix multiparticulate.

18. The process of claim 14, wherein the diffusion barrier coating is in an amount from about 0.1 to about 20 percent by weight of the substrate.

19. The process of claim 14, wherein the opioid antagonist is present in a therapeutically effective amount.

20. The process of claim 14, wherein said formulation comprises a plurality of said substrates.

21. The process of claim 14, wherein said plurality of said substrates comprises a therapeutically effective amount of said opioid antagonist.

22. The process of claim 14, wherein the coating comprising the hydrophobic material provides for the controlled release of the opioid antagonist.

23. The process of claim 14, wherein the coating comprising the hydrophobic material provides for the sequestration of the opioid antagonist.

24. The process of claim 14, wherein said opioid antagonist is selected from the group consisting of naltrexone, naloxone or pharmaceutically acceptable salts thereof.

25. The pharmaceutical formulation of claim 14, wherein the anionic polymer is a non-acrylic enteric coating material, selected from the group consisting of cellulose acetate phthalate, hydroxypropyl methylcellulose phthalate, carboxymethyl ethylcellulose, hydroxypropyl methylcellulose acetate succinate, polyvinyl acetate phthalate, cellulose acetate trimellatate, cellulose acetophthalate, cellulose acetate terephthalate, polyvinyl alcohol phthalate, and mixtures thereof.

## Patentansprüche

1. Pharmazeutische Formulierung umfassend:
ein Substrat beinhaltend einen Opiatantagonisten, wobei der Opiatantagonist protoniert ist;
eine das Substrat umhüllende, diffusionshemmende Schicht, enthaltend ein anionisches Polymer; und
eine die genannte diffusionshemmende Schicht umhüllende Schicht enthaltend eine hydrophobes Material,
wobei die diffusionshemmende Schicht in einer Menge von 0,1 bis 20 Gew.-% bezogen auf das Substrat, das den genannten Opiatantagonisten enthält, aufgetragen wird;
mit Ausnahme der folgenden Formulierungen:
A) Nonpareille Kügelchen (30/35 mesh) (39,98 mg), zuerst beschichtet mit Naltrexon HCl (2,1 mg), Hydroxypropylmethylcellulose (Opadry Clear^{®}) (0,4 mg), Natriumascorbat (0,027 mg), and Ascorbinsäure (0,05 mg), dann beschichtet mit einem Methacrylsäure-Ethylacrylatcopolymer (1:1) (Eudragit L30D^{®}) (2,164 mg), Triethylcitrat (0,433 mg), und pyrogener Kieselsäure (Cabosil^{®}) (0,108 mg), dann beschichtet mit Ammoniummethacrylatcopolymer Type B (Eudragit RS30D^{®}) (17,475 mg), Triethylcitrat (3,495 mg), und pyrogener Kieselsäure (Cabosil^{®}) (0,874 mg), und zuletzt beschichtet mit Hydroxypropylmethylcellulose (Opadry Clear^{®}) (1,899 mg) und pyrogener Kieselsäure (0,271 mg);
B) Nonpareille Kügelchen (30/35 mesh) (67,264 mg), zuerst beschichtet mit Naltrexon HCl (0,609 mg), und Hydroxypropylmethylcellulose (Opadry Clear^{®}) (0,547 mg), dann beschichtet mit einem Methacrylsäure-Ethylacrylatcopolymer (1:1) (Eudragit L30D^{®}) (2,545 mg), Triethylcitrat (0,636 mg), und Glycerinmonostearat (0,239 mg), dann beschichtet mit Ammoniummethacrylatcopolymer Type B (Eudragit RS30D^{®}) (43,789 mg), triethyl citrate (8,758 mg), und pyrogener Kieselsäure (Cabosil^{®}) (2,189 mg), und zuletzt beschichtet mit Hydroxypropylmethylcellulose (Opadry Clear^{®}) (2,053 mg) und pyrogener Kieselsäure (1,368 mg);
C) Nonpareille Kügelchen (30/35 mesh) (79,788 mg), zuerst beschichtet mit Naltrexon HCl (0,658 mg), und Hydroxypropylmethylcellulose (Opadry Clear^{®}) (0,775 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (4,062 mg);
D) Nonpareille Kügelchen (30/35 mesh) (79,788 mg), zuerst beschichtet mit Naltrexon HCl (0,658 mg), Hydroxypropylmethylcellulose (Opadry Clear^{®}) (0,775 mg), und Butylhydroxytoluol (BHT) (0,029 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit einem Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (4,062 mg);
E) Nonpareille Kügelchen (30/35 mesh) (80,179 mg), zuerst beschichtet mit Naltrexon HCl (0,584 mg), Hydroxypropylmethylcellulose (Opadry Clear^{®}) (0,341 mg), Natriumascorbat (0,065 mg), und EDTA (0,065 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (4,062 mg), und pyrogener Kieselsäure (Cabosil^{®}) (1,625 mg);
F) Nonpareille Kügelchen (30/35 mesh) (80,26 mg), zuerst beschichtet mit Naltrexon HCl (0,584 mg), Hydroxypropylmethylcellulose (Opadry Clear^{®}) (0,341 mg), und Ascorbinsäure (0,065 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (3,532 mg), und pyrogener Kieselsäure (Cabosil^{®}) (0,531 mg); und
G) Nonpareille Kügelchen (30/35 mesh) (80,211 mg), zuerst beschichtet mit Naltrexon HCl (0,61 mg), Hydroxypropylmethylcellulose (Opadry Clear^{®}) (0,919 mg), Propylgallat (0,00581 mg), und EDTA (0,00349 mg), dann beschichtet mit einem Methacrylsäure-Ethylacrylatcopolymer (1:1) (Eudragit L30D^{®}) (3,022 mg), Triethylcitrat (0,755 mg), und Glycerinmonostearat (0,29 mg), dann beschichtet mit Ammoniummethacrylatcopolymer Type B (Eudragit RS30D^{®}) (32,534 mg), Triethylcitrat (6,507 mg), und pyrogener Kieselsäure (Cabosil^{®}) (1,627 mg), und zuletzt beschichtet mit Hydroxypropylmethylcellulose (Opadry Clear^{®}) (3,538 mg), und pyrogener Kieselsäure (Cabosil^{®}) (0,529 mg).

2. Pharmazeutische Formulierung gemäß Anspruch 1, wobei das Substrat den einen Kern umhüllenden Opiatantagonisten umfasst.

3. Pharmazeutische Formulierung gemäß Anspruch 2, wobei der Kern ein pharmazeutisch akzeptables, inertes Kügelchen ist.

4. Pharmazeutische Formulierung gemäß Anspruch 1, wobei der Antagonist in einer multipartikulären Matrix dispergiert ist.

5. Pharmazeutische Formulierung gemäß Anspruch 1, wobei die diffusionshemmende Schicht in einer Menge von ungefähr 0,1 bis ungefähr 10 Gew.-% bezogen auf das Substrat vorhanden ist.

6. Pharmazeutische Formulierung gemäß Anspruch 1, wobei der Opiatantagonist in einer therapeutisch wirksamen Menge vorhanden ist.

7. Pharmazeutische Formulierung gemäß Anspruch 1, umfassend eine Mehrzahl der genannten Substrate.

8. Pharmazeutische Formulierung gemäß Anspruch 7, wobei die genannte Mehrzahl der genannten Substrate eine therapeutisch wirksame Menge des genannten Opiatantagonisten umfasst.

9. Pharmazeutische Formulierung gemäß Anspruch 1, wobei die das hydrophobe Material enthaltende Schicht die kontrollierte Freisetzung des Opiatantagonisten bewirkt.

10. Pharmazeutische Formulierung gemäß Anspruch 1, wobei die das hydrophobe Material enthaltende Schicht die Sequestrierung des Opiatantagonisten bewirkt.

11. Pharmazeutische Formulierung gemäß Anspruch 1, wobei das hydrophobe Material aus der Gruppe bestehend aus einem zellulosischem Material, einem zellulosischem Polymer, einem Acrylpolymer oder -copolymer, einem Methacrylpolymer oder -copolymer und Mischungen hiervon ausgewählt ist.

12. Pharmazeutische Formulierung gemäß Anspruch 1, wobei der genannte Opiatantagonist aus der Gruppe bestehend aus Naltrexon, Naloxon und den pharmazeutisch akzeptablen Salzen hiervon ausgewählt ist.

13. Pharmazeutische Formulierung gemäß Anspruch 1, wobei das anionische Polymer ein nicht-acrylisches, enterisches Beschichtungsmaterial ist, das aus der Gruppe bestehend aus Celluloseacetatphthalat, Hydroxypropylmethylcellulosephthalat, Carboxymethylethylcellulose, Hydroxypropylmethylcelluloseacetatsuccinat, Polyvinylacetatphthalat, Celluloseacetattrimellitat, Celluloseacetophthalat, Celluloseacetatterephthalat, Polyvinylalkoholphthalat und Mischungen hiervon ausgewählt ist.

14. Verfahren zur Herstellung einer pharmazeutischen Formulierung umfassend:
a) Herstellung eines Substrats umfassend einen Opiatantagonisten, wobei der Opiatantagonist protoniert ist;
b) Auftragung einer diffusionshemmenden Schicht umfassend ein anionisches Polymer auf das genannte Substrat; und
c) Auftragung einer die genannte diffusionshemmende Schicht umhüllenden Schicht umfassend ein hydrophobes Material,
wobei die diffusionshemmende Schicht in einer Menge von 0,1 bis 20 Gew.-% bezogen auf das Substrat, das den genannten Opiatantagonisten enthält, aufgetragen wird;
mit Ausnahme der folgenden Formulierungen:
A) Nonpareille Kügelchen (30/35 mesh) (39,98 mg), zuerst beschichtet mit Naltrexon HCl (2,1 mg), Hydroxypropylmethylcellulose (Opadry Clear^{®}) (0,4 mg), Natriumascorbat (0,027 mg), and Ascorbinsäure (0,05 mg), dann beschichtet mit einem Methacrylsäure-Ethylacrylatcopolymer (1:1) (Eudragit L30D^{®}) (2,164 mg), Triethylcitrat (0,433 mg), und pyrogener Kieselsäure (Cabosil^{®}) (0,108 mg), dann beschichtet mit Ammoniummethacrylatcopolymer Type B (Eudragit RS30D^{®}) (17,475 mg), Triethylcitrat (3,495 mg), und pyrogener Kieselsäure (Cabosil^{®}) (0,874 mg), und zuletzt beschichtet mit Hydroxypropylmethylcellulose (Opadry Clear^{®}) (1,899 mg) und pyrogener Kieselsäure (0,271 mg);
B) Nonpareille Kügelchen (30/35 mesh) (67,264 mg), uerst beschichtet mit Naltrexon HCl (0,609 mg), und Hydroxypropylmethylcellulose (Opadry Clear^{®}) (0,547 mg), dann beschichtet mit einem Methacrylsäure-Ethylacrylatcopolymer (1:1) (Eudragit L30D^{®}) (2,545 mg), Triethylcitrat (0,636 mg), und Glycerinmonostearat (0,239 mg), dann beschichtet mit Ammoniummethacrylatcopolymer Type B (Eudragit RS30D^{®}) (43,789 mg), triethyl citrate (8,758 mg), und pyrogener Kieselsäure (Cabosil^{®}) (2,189 mg), und zuletzt beschichtet mit Hydroxypropylmethylcellulose (Opadry Clear^{®}) (2,053 mg) und pyrogener Kieselsäure (1,368 mg);
C) Nonpareille Kügelchen (30/35 mesh) (79,788 mg), zuerst beschichtet mit Naltrexon HCl (0,658 mg), und Hydroxypropylmethylcellulose (Opadry Clear^{®}) (0,775 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (4,062 mg);
D) Nonpareille Kügelchen (30/35 mesh) (79,788 mg), zuerst beschichtet mit Naltrexon HCl (0,658 mg), Hydroxypropylmethylcellulose (Opadry Clear^{®}) (0,775 mg), und Butylhydroxytoluol (BHT) (0,029 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (4,062 mg);
E) Nonpareille Kügelchen (30/35 mesh) (80,179 mg), zuerst beschichtet mit Naltrexon HCl (0,584 mg), Hydroxypropylmethylcellulose (Opadry Clear^{®}) (0,341 mg), Natriumascorbat (0,065 mg), und EDTA (0,065 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (4,062 mg), und pyrogener Kieselsäure (Cabosil^{®}) (1,625 mg);
F) Nonpareille Kügelchen (30/35 mesh) (80,26 mg), zuerst beschichtet mit Naltrexon HCl (0,584 mg), Hydroxypropylmethylcellulose (Opadry Clear^{®}) (0,341 mg), und Ascorbinsäure (0,065 mg), dann beschichtet mit einem Methacrylsäure-Ethylacrylatcopolymer (1:1) (Eudragit L30D^{®}) (3,023 mg), Triethylcitrat (0,756 mg), und Glycerinmonostearat (0,284 mg), dann beschichtet mit Ammoniummethacrylatcopolymer Type B (Eudragit RS30D^{®}) (32,5 mg), Triethylcitrat (6,5 mg), und pyrogener Kieselsäure (Cabosil^{®}) (1,625 mg), und zuletzt beschichtet mit Hydroxypropylmethylcellulose (Opadry Clear^{®}) (3,532 mg), und pyrogener Kieselsäure (Cabosil^{®}) (0,531 mg); und
G) Nonpareille Kügelchen (30/35 mesh) (80,211 mg), zuerst beschichtet mit Naltrexon HCl (0,61 mg), Hydroxypropylmethylcellulose (Opadry Clear^{®}) (0,919 mg), Propylgallat (0,00581 mg), und EDTA (0,00349 mg), dann beschichtet mit einem Methacrylsäure-Ethylacrylatcopolymer (1:1) (Eudragit L30D^{®}) (3,022 mg), Triethylcitrat (0,755 mg), und Glycerinmonostearat (0,29 mg), dann beschichtet mit Ammoniummethacrylatcopolymer Type B (Eudragit RS30D^{®}) (32,534 mg), Triethylcitrat (6,507 mg), und pyrogener Kieselsäure (Cabosil^{®}) (1,627 mg), und zuletzt beschichtet mit Hydroxypropylmethylcellulose (Opadry Clear^{®}) (3,538 mg), und pyrogener Kieselsäure (Cabosil^{®}) (0,529 mg).

15. Das Verfahren gemäß Anspruch 14, wobei der genannte Opiatantagonist auf das genannte Substrat aufgetragen wird.

16. Das Verfahren gemäß Anspruch 14, wobei das Substrat ein pharmazeutisch akzeptables, inertes Kügelchen ist.

17. Das Verfahren gemäß Anspruch 14, wobei das Substrat eine multipartikuläre Matrix ist.

18. Das Verfahren gemäß Anspruch 14, wobei die diffusionshemmende Schicht in einer Menge von etwa 0,1 bis etwa 20 Gew.-% in Bezug auf das Substrat vorhanden ist.

19. Das Verfahren gemäß Anspruch 14, wobei der Opiatantagonist in einer therapeutisch wirksamen Menge vorhanden ist.

20. Pharmazeutische Formulierung gemäß Anspruch 14, wobei die genannte Formulierung eine Mehrzahl der genannten Substrate beinhaltet.

21. Pharmazeutische Formulierung gemäß Anspruch 14, wobei die genannte Mehrzahl der genannten Substrate eine therapeutisch wirksame Menge des genannten Opiatantagonisten umfasst.

22. Pharmazeutische Formulierung gemäß Anspruch 14, wobei die das hydrophobe Material enthaltende Schicht die kontrollierte Freisetzung des Opiatantagonisten bewirkt.

23. Pharmazeutische Formulierung gemäß Anspruch 14, wobei die das hydrophobe Material enthaltende Schicht die Sequestrierung des Opiatantagonisten bewirkt.

24. Pharmazeutische Formulierung gemäß Anspruch 14, wobei der genannte Opiatantagonist aus der Gruppe bestehend aus Naltrexon, Naloxon und den pharmazeutisch akzeptablen Salzen hiervon ausgewählt ist.

25. Pharmazeutische Formulierung gemäß Anspruch 14, wobei das anionische Polymer ein nicht-acrylisches, enterisches Beschichtungsmaterial ist, das aus der Gruppe bestehend aus Celluloseacetatphthalat, Hydroxypropylmethylcellulosephthalat, Carboxymethylethylcellulose, Hydroxypropylmethylcelluloseacetatsuccinat, Polyvinylacetatphthalat, Celluloseacetattrimellitat, Celluloseacetophthalat, Celluloseacetatterephthalat, Polyvinylalkoholphthalat und Mischungen hiervon ausgewählt ist.

## Revendications

1. Formulation pharmaceutique qui comprend :
un substrat comprenant un antagoniste des opiacés, l'antagoniste des opiacés étant protoné ;
un revêtement formant barrière de diffusion comprenant un polymère anionique revêtu sur ledit substrat ; et
un revêtement comprenant un matériau hydrophobe revêtu sur ledit revêtement formant barrière de diffusion, le revêtement formant barrière de diffusion étant appliqué en une quantité comprise entre 0,1 et 20 % en poids du substrat comprenant ledit antagoniste des opiacés,
à l'exception des formulations suivantes :
A) des billes nonpareilles (taille 30/35) (39,98 mg), d'abord revêtues de naltrexone HCl (2,1 mg), d'hydroxypropylméthyl cellulose (Opadry Clear®) (0,4 mg), d'ascorbate de sodium (0,027 mg), et d'acide ascorbique (0,05 mg), puis revêtues d'un copolymère acide méthacrylique - acrylate d'éthyle (1/1) (Eudragit L30D®) (2,164 mg), de citrate de triéthyle (0,433 mg), et de silice fumée (Cabosil®) (0,108 mg), puis revêtues d'un copolymère d'ammonio méthacrylate Type B (Eudragit RS30D®) (17,475 mg), de citrate de triéthyle (3,495 mg), et de silice fumée (Cabosil®) (0,874 mg), et finalement revêtues d'hydroxypropylméthylcellulose (Opadry Clear®) (1,899 mg) et de silice fumée (0,271 mg) ;
B) des billes nonpareilles (taille 30/35) (67,264 mg), d'abord revêtues de naltrexone HCl (0,609 mg), et d'hydroxypropylméthylcellulose (Opadry Clear®) (0,547 mg), puis revêtues d'un copolymère acide méthacrylique - acrylate d'éthyle (1/1) (Eudragit L30D®) (2,545 mg), de citrate de triéthyle (0,636 mg), et de monostéarate de glycéryle (0,239 mg), puis revêtues d'un copolymère d'ammonio méthacrylate Type B (Eudragit RS30D®) (43,789 mg), de citrate de triéthyle (8,758 mg), et de silice fumée (Cabosil®) (2,189 mg), et finalement revêtues d'hydroxypropylméthylcellulose (Opadry Clear®) (2,053 mg) et de silice fumée (1,368 mg) ;
C) des billes nonpareilles (taille 30/35) (79,788 mg), d'abord revêtues de naltrexone HCl (0,658 mg), et d'hydroxypropylméthylcellulose (Opadry Clear®) (0,775 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (4,062 mg) ;
D) des billes nonpareilles (taille 30/35) (79,788 mg), d'abord revêtues de naltrexone HCl (0,658 mg), d'hydroxypropylméthylcellulose (Opadry Clear®) (0,775 mg), et d'hydroxytoluène butylé (BHT) (0,029 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (4,062 mg) ;
E) des billes nonpareilles (taille 30/35) (80,179 mg), d'abord revêtues de naltrexone HCl (0,584 mg), d'hydroxypropylméthylcellulose (Opadry Clear®) (0,341 mg), d'ascorbate de sodium (0,065 mg), et d'EDTA (0,065 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (4,062 mg), et de silice fumée (Cabosil®) (1,625 mg) ;
F) des billes nonpareilles (taille 30/35) (80,26 mg), d'abord revêtues de naltrexone HCl (0,584 mg), d'hydroxypropylméthylcellulose (Opadry Clear®) (0,341 mg), et d'acide ascorbique (0,065 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (3,532 mg), et de silice fumée (Cabosil®) (0,531 mg) ; et
G) des billes nonpareilles (taille 30/35) (80,211 mg), d'abord revêtues de naltrexone HCl (0,61 mg), d'hydroxypropylméthylcellulose (Opadry Clear®) (0,919 mg), de gallate de propyle (0,00581 mg), et d'EDTA (0,00349 mg), puis revêtues d'un copolymère acide méthacrylique - acrylate d'éthyle (1/1) (Eudragit L30D®) (3,022 mg), de citrate de triéthyle (0,755 mg), et de monostéarate de glycéryle (0,29 mg), puis revêtues d'un copolymère d'ammonio méthacrylate Type B (Eudragit RS30D®) (32,534 mg), de citrate de triéthyle (6,507 mg), et de silice fumée (Cabosil®) (1,627 mg), et finalement revêtues d'hydroxypropylméthylcellulose (Opadry Clear®) (3,538 mg), et de silice fumée (Cabosil®) (0,529 mg).

2. Formulation pharmaceutique selon la revendication 1, dans laquelle le substrat comprend l'antagoniste des opiacés revêtu sur un noyau.

3. Formulation pharmaceutique selon la revendication 2, dans laquelle le noyau est une bille inerte pharmaceutiquement acceptable.

4. Formulation pharmaceutique selon la revendication 1, dans laquelle l'antagoniste est dispersé dans des multiparticules matricielles.

5. Formulation pharmaceutique selon la revendication 1, dans laquelle le revêtement formant barrière de diffusion est présent en une quantité d'environ 0,1 à environ 10 % en poids du substrat.

6. Formulation pharmaceutique selon la revendication 1, dans laquelle l'antagoniste des opiacés est présent en une quantité thérapeutiquement efficace.

7. Formulation pharmaceutique selon la revendication 1, qui comprend une pluralité desdits substrats.

8. Formulation pharmaceutique selon la revendication 7, dans laquelle ladite pluralité desdits substrats comprend une quantité thérapeutiquement efficace dudit antagoniste des opiacés.

9. Formulation pharmaceutique selon la revendication 1, dans laquelle le revêtement comprenant le matériau hydrophobe permet la libération contrôlée de l'antagoniste des opiacés.

10. Formulation pharmaceutique selon la revendication 1, dans laquelle le revêtement comprenant le matériau hydrophobe permet la séquestration de l'antagoniste des opiacés.

11. Formulation pharmaceutique selon la revendication 1, dans laquelle le matériau hydrophobe est choisi dans le groupe constitué par un matériau cellulosique, un polymère cellulosique, un polymère ou copolymère acrylique, un polymère ou copolymère méthacrylique, et leurs mélanges.

12. Formulation pharmaceutique selon la revendication 1, dans laquelle ledit antagoniste des opiacés est choisi dans le groupe constitué par la naltrexone, la naloxone et leurs sels pharmaceutiquement acceptables.

13. Formulation pharmaceutique selon la revendication 1, dans laquelle le polymère anionique est un matériau d'enrobage gastrorésistant non acrylique, choisi dans le groupe constitué par l'acétate phtalate de cellulose, le phtalate d'hydroxypropylméthylcellulose, la carboxyméthyléthylcellulose, l'acétate succinate d'hydroxypropylméthylcellulose, l'acétate phtalate de polyvinyle, l'acétate trimellatate de cellulose, l'acétophtalate de cellulose, l'acétate téréphtalate de cellulose, le phtalate d'alcool polyvinylique, et leurs mélanges.

14. Procédé de préparation d'une formulation pharmaceutique qui comprend :
a) la formation d'un substrat comprenant un antagoniste des opiacés, l'antagoniste des opiacés étant protoné ;
b) l'application d'un revêtement formant barrière de diffusion comprenant un polymère anionique sur ledit substrat ; et
c) l'application d'un revêtement comprenant un matériau hydrophobe sur ledit revêtement formant barrière de diffusion, le revêtement formant barrière de diffusion étant appliqué en une quantité comprise entre 0,1 et 20 % en poids du substrat comprenant ledit antagoniste des opiacés,
à l'exception d'un procédé de préparation de l'une des formulations suivantes :
A) des billes nonpareilles (taille 30/35) (39,98 mg), d'abord revêtues de naltrexone HCl (2,1 mg), d'hydroxypropylméthylcellulose (Opadry Clear®) (0,4 mg), d'ascorbate de sodium (0,027 mg), et d'acide ascorbique (0,05 mg), puis revêtues d'un copolymère acide méthacrylique - acrylate d'éthyle (1/1) (Eudragit L30D®) (2,164 mg), de citrate de triéthyle (0,433 mg), et de silice fumée (Cabosil®) (0,108 mg), puis revêtues d'un copolymère d'ammonio méthacrylate Type B (Eudragit RS30D®) (17,475 mg), de citrate de triéthyle (3,495 mg), et de silice fumée (Cabosil®) (0,874 mg), et finalement revêtues d'hydroxypropylméthylcellulose (Opadry Clear®) (1,899 mg) et de silice fumée (0,271 mg) ;
B) des billes nonpareilles (taille 30/35) (67,264 mg), d'abord revêtues de naltrexone HCl (0,609 mg), et d'hydroxypropylméthylcellulose (Opadry Clear®) (0,547 mg), puis revêtues d'un copolymère acide méthacrylique - acrylate d'éthyle (1/1) (Eudragit L30D®) (2,545 mg), de citrate de triéthyle (0,636 mg), et de monostéarate de glycéryle (0,239 mg), puis revêtues d'un copolymère d'ammonio méthacrylate Type B (Eudragit RS30D®) (43,789 mg), de citrate de triéthyle (8,758 mg), et de silice fumée (Cabosil®) (2,189 mg), et finalement revêtues d'hydroxypropylméthylcellulose (Opadry Clear®) (2,053 mg) et de silice fumée (1,368 mg) ;
C) des billes nonpareilles (taille 30/35) (79,788 mg), d'abord revêtues de naltrexone HCl (0,658 mg), et d'hydroxypropylméthylcellulose (Opadry Clear®) (0,775 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (4,062 mg) ;
D) des billes nonpareilles (taille 30/35) (79,788 mg), d'abord revêtues de naltrexone HCl (0,658 mg), d'hydroxypropylméthylcellulose (Opadry Clear®) (0,775 mg), et d'hydroxytoluène butylé (BHT) (0,029 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (4,062 mg) ;
E) des billes nonpareilles (taille 30/35) (80,179 mg), d'abord revêtues de naltrexone HCl (0,584 mg), d'hydroxypropylméthylcellulose (Opadry Clear®) (0,341 mg), d'ascorbate de sodium (0,065 mg), et d'EDTA (0,065 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (4,062 mg), et de silice fumée (Cabosil®) (1,625 mg) ;
F) des billes nonpareilles (taille 30/35) (80,26 mg), d'abord revêtues de naltrexone HCl (0,584 mg), d'hydroxypropylméthylcellulose (Opadry Clear®) (0,341 mg), et d'acide ascorbique (0,065 mg), puis revêtues d'un copolymère acide méthacrylique - acrylate d'éthyle (1/1) (Eudragit L30D®) (3,023 mg), de citrate de triéthyle (0,756 mg), et de monostéarate de glycéryle (0,284 mg), puis revêtues d'un copolymère d'ammonio méthacrylate Type B (Eudragit RS30D®) (32,5 mg), de citrate de triéthyle (6,5 mg), et de silice fumée (Cabosil®) (1,625 mg), et finalement revêtues d'hydroxypropylméthylcellulose (Opadry Clear®) (3,532 mg), et de silice fumée (Cabosil®) (0,531 mg) ; et
G) des billes nonpareilles (taille 30/35) (80,211 mg), d'abord revêtues de naltrexone HCl (0,61 mg), d'hydroxypropylméthylcellulose (Opadry Clear®) (0,919 mg), de gallate de propyle (0,00581 mg), et d'EDTA (0,00349 mg), puis revêtues d'un copolymère acide méthacrylique - acrylate d'éthyle (1/1) (Eudragit L30D®) (3,022 mg), de citrate de triéthyle (0,755 mg), et de monostéarate de glycéryle (0,29 mg), puis revêtues d'un copolymère d'ammonio méthacrylate Type B (Eudragit RS30D®) (32,534 mg), de citrate de triéthyle (6,507 mg), et de silice fumée (Cabosil®) (1,627 mg), et finalement revêtues d'hydroxypropylméthylcellulose (Opadry Clear®) (3,538 mg), et de silice fumée (Cabosil®) (0,529 mg).

15. Procédé selon la revendication 14, dans lequel ledit antagoniste des opiacés est appliqué sur ledit substrat.

16. Procédé selon la revendication 14, dans lequel le substrat est une bille inerte pharmaceutiquement acceptable.

17. Procédé selon la revendication 14, dans lequel le substrat est une multiparticule matricielle.

18. Procédé selon la revendication 14, dans lequel le revêtement formant barrière de diffusion est présent en une quantité d'environ 0,1 à environ 20 % en poids du substrat.

19. Procédé selon la revendication 14, dans lequel l'antagoniste des opiacés est présent en une quantité thérapeutiquement efficace.

20. Procédé selon la revendication 14, dans lequel ladite formulation comprend une pluralité desdits substrats.

21. Procédé selon la revendication 14, dans lequel ladite pluralité desdits substrats comprend une quantité thérapeutiquement efficace dudit antagoniste des opiacés.

22. Procédé selon la revendication 14, dans lequel le revêtement comprenant le matériau hydrophobe permet la libération contrôlée de l'antagoniste des opiacés.

23. Procédé selon la revendication 14, dans lequel le revêtement comprenant le matériau hydrophobe permet la séquestration de l'antagoniste des opiacés.

24. Procédé selon la revendication 14, dans lequel l'antagoniste des opiacés est choisi dans le groupe constitué par la naltrexone, la naloxone ou leurs sels pharmaceutiquement acceptables.

25. Formulation pharmaceutique selon la revendication 14, dans laquelle le polymère anionique est un matériau d'enrobage gastrorésistant non acrylique, choisi dans le groupe constitué par l'acétate phtalate de cellulose, le phtalate d'hydroxypropylméthylcellulose, la carboxyméthyléthylcellulose, l'acétate succinate d'hydroxypropylméthylcellulose, l'acétate phtalate de polyvinyle, l'acétate trimellatate de cellulose, l'acétophtalate de cellulose, l'acétate téréphtalate de cellulose, le phtalate d'alcool polyvinylique, et leurs mélanges.
